# EUROPÄISCHE PATENTSCHRIFT

(11) **EP 2 468 874 B1**
(45) Veröffentlichungstag und Bekanntmachung des Hinweises auf die Patenterteilung: **25.06.2014**
(21) Anmeldenummer: 10196776.8
(22) Anmeldetag: 23.12.2010
(51) Int. Cl.: C12P 5/00, C12P 5/02, C12P 7/02, C12P 7/10, C12P 7/16, C12P 7/24, C12P 7/28, C12P 7/40, B01D 3/00, C07C 29/76, B01D 53/02, B01J 29/06, B01J 29/40

(54) **Verfahren zur Herstellung organischer Verbindungen via Fermentation von Biomasse und Zeolith-Katalyse**
Process for producing organic compounds via fermentation of biomass and zeolite catalysis
Procédé de production des composés organiques via fermentation de biomasse et catalyse zéolitique

(43) Veröffentlichungstag der Anmeldung: 27.06.2012
(73) Patentinhaber: Clariant Produkte (Deutschland) GmbH, 65929 Frankfurt/Main (DE)
(72) Erfinder: Zavrel, Michael, Dr., 81379 München (DE); Franke, Oliver, Dr., 81671 München (DE); Richter, Oliver, Dr., 82110 Germering (DE); Kraus, Michael, Dr., 82178 Puchheim (DE)
(74) Vertreter: HOFFMANN EITLE

(56) Entgegenhaltungen:
- WO-A1-2007/137566
- WO-A1-2008/066581
- WO-A1-2010/037635
- US-A- 4 621 164

## Beschreibung

### Gebiet der Erfindung

Die Erfindung betrifft ein Verfahren zur Herstellung von organischen Verbindungen aus Biomasse.

### Stand der Technik

Aus der Literatur sind Prozesse bekannt, welche die Herstellung von organischen Verbindungen aus fermentativ gewonnenem Alkohol ermöglichen. Sie bestehen grundlegend aus den Schritten Zucker-Fermentation, Destillation des Fermentationsmediums, katalytische Umsetzung des thermisch abgetrennten Alkohols zu organischen Verbindungen, und Abtrennung der organischen Verbindungen vom Prozesswasser (siehe z.B. US 3936353; CA 2360981).

Abweichend hierzu kann gemäß Patentschrift US 4690903 der fermentativ gewonnene Alkohol durch Sorption an einen Adsorber aus der Fermentationsbrühe gewonnen werden, wobei die Sorption direkt in der Fermentationsbrühe erfolgt. Im Falle eines Zeolithen als Adsorber ist es optional möglich, den beladenen Zeolithen in eine Reaktionszone zu überführen, in der der sorbierte Alkohol katalytisch mit dem Zeolithen zu organischen Verbindungen umgesetzt wird.

Eine Umsetzung von Alkoholen zu organischen verbindungen mit einem niedrigeren Sauerstoff-zu-Kohlenstoff-Verhältnis eignen sich insbesondere Dehydratisierungsreaktionen. Für diese Dehydratisierung von Alkoholen (meist Ethanol) sind in der Literatur Zeolithe vom Typ MFI in der H-Form (H-ZSM-5, SiO₂/Al₂O₃>10) als Katalysator beschrieben (siehe z.B. US 3936353; US 4690903; US 4621164; Oudejans et al., App. Catalysis Vol. 3, 1982, S.109; Aguayo et al., J. Chem. Technol. Biotechnol. Vol. 77, 2002, S.211). Weiterhin sind auch Modifikationen des Zeolithen H-ZSM-5 durch z.B. Imprägnierung mit Metallen/Metalloxiden oder Phosphorsäure bekannt, durch die die Selektivität der Umsetzung zu Ethen (US 4698452) oder auch die Selektivität der Umsetzung zu Aromaten (WO 2007/137566 A1) beeinflusst werden kann. Neben H-ZSM-5 Zeolithen sind als weitere Katalysatoren für die Ethanol-Dehydratisierung auch andere Zeolith-Typen (US 4621164; Oudejans et al., App. Catalysis Vol. 3, 1982, S.109), mesoporöse Molekularsiebe (Varisli et al.; Chem. Eng. Sci. Vol. 65, 2010, S.153) und Hydroxyapatite (Tsuchida et al., Ind. Eng. Chem. Res. Vol. 47; 2008, S.1443) untersucht worden.

Gemäß dem Stand der Technik erfolgt die Dehydratisierung in einem Festbettreaktor bei Temperaturen zwischen 150°C und 500°C, Absolutdrücken von 1 bar bis 100 bar und liquid-hourly-space-velocities (LHSV = Volumenstrom flüssiges Edukt / Volumen Katalysator) im Bereich von 0,5 h⁻¹ bis 50 h⁻¹ (siehe z.B. US 4621164; Oudejans et al., App. Catalysis Vol. 3, 1982, S.109).

Durch Versetzen des Ethanol-Eingangsstroms mit Wasser kann der Aromatenanteil im Produktstrom vergrößert und die Katalysatordeaktivierung aufgrund Verkokung reduziert werden (Oudejans et al., App. Catalysis Vol. 3, 1982, S.109). Die Ausbeute an flüssigen organischen Verbindungen kann ebenfalls durch Variation des Wasseranteils beeinflusst werden (US 4621164). Ein niedriger Wasseranteil bedingt einen hohen Anteil der organischen Verbindungen und umgekehrt.

WO 2008/066581 A1 beschreibt ein Verfahren zum Herstellen von mindestens einem Buten aus wässrigem 2-Butanol in Gegenwart von Wasser; Butanol und Wasser werden dafür miteinander umgesetzt. Das Butanol/Wasser-Reagenz kann optional aus Fermentationsbrühe stammen. Es wird weiter vorgeschlagen, dass die geklärte Fermentationsbrühe zum Beispiel durch Gas-Stripping behandelt wird. Dabei wird ein angereichertes Trägergas erzeugt, das 2-Butanol und Wasser umfasst. Dieses angereicherte Trägergas kann entweder direkt der Umsetzung zu Kohlenwasserstoffen eingesetzt werden, oder einer Destillation unterzogen werden, so dass ein wässriger 2-Butanol-Strom erhalten wird, der zur Umsetzung zu Kohlenwasserstoffen eingesetzt werden kann. Allerdings wird keine Desorption flüchtiger organischer Verbindungen im Vorfeld der katalytischen Umsetzung gelehrt.

WO 2010/037635 A1 beschreibt ein Gas-Stripping-Verfahren mit der Adsorption an einen Adsorber sowie der Desorption davon. Die Desorption dient dem Ziel, den Adsorber wieder verwenden zu können. Es wird aber nicht gelehrt, welche Maßnahmen zu ergreifen seien, um eine evtl. nachgeschaltete katalytische Umsetzung zu verbessern.

Nachteilig bei allen dem Stand der Technik entsprechenden Verfahren zur Herstellung von organischen Verbindungen aus Zuckern ist, dass flüchtige Fermentationsnebenprodukte (z.B. Furane) sowie bei der Fermentation üblicherweise eingesetzte flüchtige Zusatzstoffe (z.B. Ammoniak als pH-Stellmittel) nicht selektiv abgetrennt werden können. Diese führen bei der nachgeschalteten katalytischen Umsetzung zu einer Deaktivierung des (Zeolith-)Katalysators und somit zu einer Verringerung der Katalysatoraktivität und Selektivität (siehe z.B. Hutchings, Studies in Surface Sience and Catalysis Vol. 61, 1991, S.405).

Ebenso ist es von Nachteil, dass gemäß dem Stand der Technik die zur Herstellung des Alkohols notwendige Fermentation nicht direkt mit der katalytischen Umsetzung gekoppelt werden kann. Die Fermentation wird jedoch bei höheren Konzentrationen des Zwischenproduktes Alkohol in der Regel inhibiert, wodurch die Ausbeute und die Produktivität (Raum-Zeit-Ausbeute) an organischen Verbindungen begrenzt wird. Beispielsweise zeigen Dominguez et al. (Biotech. Bioeng., 2000, Vol. 67, S. 336-343), dass die Umsetzung von C5-Zuckern zu Ethanol mit der Hefe *Pichia* stipitis bei nur 2% (w/v) Ethanol inhibiert wird. Ebenso ist bei der Fermentation mit Clostridien zu Aceton, Butanol und Ethanol ein inhibierender und zunehmend toxischer Einfluss der gebildeten Produkte zu beobachten, so dass Butanol-Konzentrationen von 1,5% (w/v) in der Regel nicht übertroffen werden (Häggström L., Biotech. Advs., 1985, Vol. 3, S. 13-28).

Bei Einsatz von Zeolithen zur Sorption des Alkohols im Fermentationsmedium ist zusätzlich von Nachteil, dass das sorptionsverhalten des Zeolithen mit zunehmender Standzeit aufgrund von Fouling-Prozessen nachlässt. Ebenso ist die Abtrennung des Zeolithen von weiteren im Fermentationsmedium enthaltenen Feststoffen (z.B. Zellen, Stoffwechselnebenprodukte, Nährmedienbestandteile) technisch aufwändig. Desweiteren ist bei den im Stand der Technik beschriebenen thermischen Trennverfahren zur Abtrennung des Alkohols aus dem Fermentationsmedium von Nachteil, dass bei einer einfachen Destillation die Zusammensetzung des Destillatstromes durch die Ausgangskonzentration und durch das thermodynamische stoffliche Gleichgewicht beschränkt ist. Durch Anwendung einer mehrfachen Destillation bzw. Rektifikation kann die Zusammensetzung des Destillatstromes variiert werden. Jedoch ist hier besonders von Nachteil, dass aufgrund der verfahrensbedingten mehrfachen Kondensation des Destillates ein höherer Energieeintrag notwendig wird.

### zusammenfassung der Erfindung

Vor diesem Hintergrund besteht die Aufgabe der vorliegenden Erfindung darin, ein wirtschaftliches Verfahren zur Herstellung von organischen Verbindungen aus Biomasse zu entwickeln, welches die Nachteile des Standes der Technik beseitigt und eine hohe Ausbeute an organischen Verbindungen bei möglichst geringem apparativem Aufwand erlaubt.

Dieses Problem konnte überraschenderweise durch die Kombination einer Fermentation mit Produktabtrennung über Gas-Stripping, Adsorption, Desorption und katalytischer Umsetzung gelöst werden, wodurch eine Umwandlung von Biomasse in organische Verbindungen möglich ist, und wobei alle Verfahrensschritte parallel ablaufen können.

Es wird somit erfindungsgemäß ein zur Herstellung von organischen Verbindungen bereitgestellt, das die folgenden Schritte umfasst:
a. die fermentative Umsetzung von Biomasse zu flüchtigen organischen Verbindungen in einem Bioreaktor;
b. die Entfernung der flüchtigen organischen Verbindungen durch Gas-Stripping mit Hilfe eines Trägergases;
c. die Adsorption der flüchtigen organischen Verbindungen aus dem Gasstrom;
d. die Desorption der adsorbierten flüchtigen organischen Verbindungen von dem Adsorber;
e. die katalytische Umsetzung der flüchtigen organischen Verbindungen.

In Verfahrensschritt d liegt der Anteil flüchtiger organischer Verbindungen in dem Desorbatstrom bevorzugt zwischen 10 % (w/w) und 90 % (w/w), besonders bevorzugt zwischen 30 %(w/w) und 70 % (w/w) und noch bevorzugter zwischen 35 % (w/w) und 60 %(w/w).

Die Produkte der katalytischen Umsetzung können anschließend beispielsweise durch Kondensation des Produktstroms und Phasentrennung, vorzugsweise über Dekantation, aufgearbeitet werden.

### Detaillierte Beschreibung der Erfindung

Im Rahmen dieser Erfindung wird ein verfahren zur Herstellung von organischen Verbindungen bereitgestellt, das die folgenden Verfahrensschritte umfasst:
a. die fermentative Umsetzung von Biomasse zu flüchtigen organischen Verbindungen in einem Bioreaktor;
b. die Entfernung der flüchtigen organischen Verbindungen durch Gas-Stripping mit Hilfe eines Trägergases;
c. die Adsorption der flüchtigen organischen Verbindungen aus dem Gasstrom;
d. die Desorption der adsorbierten flüchtigen organischen Verbindungen von dem Adsorber;
e. die katalytische Umsetzung der flüchtigen organischen Verbindungen.

Die einzelnen Verfahrensschritte sind im folgenden näher beschrieben:

### a. Fermentation

Für die Fermentation wird eine Lösung mit Biomasse bereitgestellt. Unter Biomasse wird hierbei biologisches Material verstanden, welches eine oder mehrere der folgenden Kompontenten umfasst: Cellulose, Hemicellulose, Lignin, Pektin, Stärke, Saccharose, Chitin, Proteine und andere Biopolymere, sowie Fette und Öle. Außerdem schließt dieser Begriff auch biologische Materialien ein, die Zucker, insbesondere C5- und C6-Zucker, Aminosäuren, Fettsäuren und andere biologische Monomere enthalten bzw. aus denen diese Monomere gewonnen werden können, vorzugsweise durch Hydrolyse. Vorzugsweise enthält die Lösung zu Beginn der Fermentation weniger als 200 g/L Zucker, besonders bevorzugt weniger als 100 g/L Zucker. In einer bevorzugten Ausführungsform enthält die Lösung Zucker, welche aus lignocellulosischer Biomasse gewonnen werden, besonders bevorzugt werden diese durch vorhergehende enzymatische Hydrolyse gewonnen. Eine ebenfalls bevorzugte Verfahrensweise ist die Kombination der Fermentation mit der enzymatischen Hydrolyse, so dass die Hydrolyse und Fermentation simultan erfolgen. Das heißt, falls wie in der weiter unten beschriebenen bevorzugten Ausführungsform die Fermentation zeitgleich mit den nachfolgenden Schritten abläuft, auch eine Kombination dieser Ausführungsformen möglich ist, das heißt, dass sowohl die Hydrolyse als auch die Fermentation zeitgleich mit den nachfolgenden Schritten ablaufen.

In einer weiteren bevorzugten Ausführungsform enthält die Fermentationslösung eine oder mehrere niedermolekulare Kohlenstoffquellen, sowie optional eine oder mehrere niedermolekulare Stickstoffquellen. Bevorzugte niedermolekulare Kohlenstoffquellen, sind Monosaccharide wie Glucose, Fructose, Galactose, Xylose, Arabinose, Mannose, Dissacchairde wie Saccharose, Lactose, Maltose, Cellobiose, Zuckersäuren wie Galacturonsäure, Gluconsäure, Polyole wie Glycerin, Sorbitol, sowie Öle, Fette und Fettsäuren. Bevorzugte Stickstoffquellen sind Ammoniak, Ammoniumsalze, Nitratsalze, Aminosäuren, Harnstoff und Proteinhydrolysate. Unter niedermolekular wird verstanden, dass das Molekulargewicht bevorzugt weniger als 2500, und besonders bevorzugt weniger als 1000 beträgt. Ammoniak ist dabei besonders als Stickstoffquelle zu bevorzugen, da dieser zugleich als pH-Stellmittel dient, d.h. zugesetzt werden kann, falls der pH-Wert vor der Fermentation zu niedrig liegt. Außerdem kann Ammoniak in einer besonderen Ausführungsform auch während der Fermentation zugesetzt werden, falls durch die Stoffwechselaktivität der fermentierten Mikroorganismen der pH-Wert abfällt. Hierdurch kann der pH über die gesamte Dauer der Fermentation eingestellt bzw. reguliert werden. Der Fermentationslösung können neben Mikroorganismen und Enzymen weitere Zusatzstoffe wie andere pH-Stellmittel und Antischaummittel zugesetzt werden. Als Mikroorganismen eignen sich Hefen, Pilze und/oder Bakterien. Bevorzugt sind Mikroorganismen, die Alkohole, Ketone, Aldehyde und/oder organische Säuren bilden. Besonders bevorzugte Produkte sind leicht flüchtige organische Verbindungen wie Ethanol und/oder Aceton und/oder Butanole. Als flüchtige Verbindung wird hierbei eine Verbindung verstanden, die bei 20 °C einen Dampfdruck von mehr als 1,0 hPa, bevorzugt mehr als 5,0 hPa hat. Dies schließt Verbindungen ein, die bei 20 °C einen Dampfdruck gleich oder größer dem des 1-Butanols haben, wie zum Beispiel 2-Butanol, tertiär-Butanol, Ethanol, 1-Propanol, Isopropanol und Aceton. Das heißt, in einer bevorzugten Ausführungsform umfasst die vorliegende Erfindung ein Verfahren, das weiterhin dadurch charakterisiert ist, dass es sich bei den flüchtigen organischen Verbindungen um Alkohole und/oder Ketone und/oder Aldehyde und/oder organische Säuren, bevorzugt um Ethanol und/oder Butanol und/oder Aceton handelt. Butanol, soweit nicht weiter spezifiziert, schließt alle Butanole ein, besonders bevorzugt ist jedoch 1-Butanol.

Die Fermentation erfolgt typischerweise bei Temperaturen zwischen 10 und 70 °C, bevorzugt zwischen 20 und 60 °C, besonders bevorzugt zwischen 30 und 50 °C. Die Fermentation erfolgt vorzugsweise im Batch-Betrieb. In einer weiteren bevorzugten Ausführungsform wird Nährmedium kontinuierlich während der Fermentation zugeführt (Fed-Batch-Betrieb). Weiterhin bevorzugt ist ein kontinuierlicher Betrieb der Fermentation. Weiterhin bevorzugt sind die Betriebsweisen repeated-batch und repeated-fed-batch, sowie zweistufige Verfahrensweisen und Kaskaden.

Die Fermentation kann durch isolierte Enzyme, die zur Fermentationslösung gegeben werden, durchgeführt werden. Bevorzugt ist aber, dass die Fermentation mit Hilfe mindestens eines Mikroorganismus durchgeführt wird. Dieser mindestens eine Mikroorganismus wird bevorzugt ausgewählt aus mesophilen und thermophilen Organismen. Die mesophilen sowie die thermophile Organismen können wiederum ausgewählt werden aus der Gruppe bestehend aus Bakterien, Archaen und Eukaryonten, wobei bei Eukaryonten besonders Pilze, und ganz besonders Hefen bevorzugt sind. Es werden ganz besonders bevorzugt mesophile Hefen verwendet, wie beispielsweise Saccharomyces cerevisiae, Pichia *stipitis*, *Pichia segobiensis*, *Candida shehatae*, *Candida tropicalis, Candida boidinii*, *Candida tenuis*, *Pachysolen tatanophilus, Hansenula polymorpha, Candida famata, Candida parapsilosis, Candida rugosa*, *Candida sonorensis*, *Issatchenkia terricola, Kloeckera apis, Pichia barkeri*, *Pichia cactophila*, *Pichia deserticola*, *Pichia norvegensis, Pichia membranaefaciens*, *Pichia Mexicana und Torulaapora delbrueckii.* Mesophile Bakterien sind beispielsweise Clostridium acetobutylicum, Clostridium beijerincki, Clostridium saccharobutylicum, Clostridium saccharoperbutylacetonicum, Escherichia coli, Zymomonas mobilis. In einer alternativen besonders bevorzugten Ausführung werden thermophile Organismen verwendet. Thermophile Hefen sind beispielsweise *Candida bovina*, *Candida picachoensis, Candida emberorum, Candida pintolopesii, Candida thermophila*, *Kluyveromyces marxianus*, *Kluyveromyces fragilis*, *Kazachstania telluris*, *Issatchenkia orientalis und Lachancea thermolerans*. *Thermophile* Bakterien sind unter anderem *Clostridium thermocellum*, *Clostridium thermohydrosulphuricum, Clostridium thermosaccharolyticium, Thermoanaerabium brockii*, *Thermobacteroides acetoethylicus*, *Thermoanaerobacter ethanolicus*, *Clostridium thermoaceticum*, Clostridium *thermoautotrophicum, Acetogenium kivui, Desulfotomaculum nigrificans,* und *Desulfovibrio thermophilus*, *Thermoanaerobacter tengcongensis*, *Bacillus stearothermophilus und Thermoanaerobacter mathranii.* In einer alternativen, weiter bevorzugten Ausführungsform werden Mikroorganismen verwendet, die mittels genetischer Methoden verändert wurden.

### b. Gas-Stripping

Gemäß der vorliegenden Erfindung erfolgt eine Überführung der flüchtigen Bestandteile, insbesondere der flüchtigen organischen Produkte, durch Strippen mit einem Trägergas in die Gasphase. Beim Gas-Stripping, auch Austreiben genannt, werden der flüssigen Phase durch das Durchleiten von Gas flüchtige Verbindungen entzogen und in die gasförmige Phase überführt. Diese Überführung kann in einer bevorzugten Ausführungsform kontinuierlich erfolgen. Kontinuierliche Entfernung der flüchtigen Bestandteile bezeichnet dabei die Entfernung der flüchtigen Bestandteile durch Gas-Stripping, parallel zu deren fermentativer Herstellung. Als Trägergas kommen inerte Gase wie beispielsweise Kohlendioxid, Helium, Wasserstoff, Stickstoff oder Luft in Frage, sowie Mischungen dieser Gase. Als inert gelten dabei Gase, die sehr reaktionsträge sind, sich also nur an wenigen chemischen Reaktionen beteiligen können. Besonders bevorzugt sind Kohlendioxid und Mischungen aus Kohlendioxid und Luft, wodurch bei Bedarf mikroaerobe Bedingungen eingestellt werden können. Ein Vorteil des erfindungsgemäßen Verfahrens besteht darin, dass die während der Fermentation gebildeten Fermentationsabgase direkt als Trägergas verwendet werden können. Es ist also in einer besonderen Ausführungsform bevorzugt, dass die Fermentationsabgase als Trägergas eingesetzt werden.

Entsprechend des erfindungsgemäßen Verfahrens erfolgt die Fermentation und das Gas-Stripping in einem Reaktor, der bevorzugt ausgewählt wird aus der Gruppe bestehend aus einem Rührkesselreaktor, einem Schlaufenreaktor, einem Air-Lift-Reaktor oder einem Blasensäulenreaktor. Besonders bevorzugt ist die Dispergierung der Gasblasen, die beispielsweise durch einen Sparger und/oder einen geeigneten Rührer erzielt wird. Zudem ist das Gas-Stripping über eine mit dem Bioreaktor verbundene, externe Gas-Stripping-Säule möglich, die optional kontinuierlich mit der Fermentationslösung gespeist wird und deren Auslass wieder zurück in den Bioreaktor geführt werden kann. Besonders bevorzugt wird eine solche externe Gas-Stripping-Säule im Gegenstrom und/oder in Kombination mit Füllkörpern, bevorzugterweise mit Raschig-Ringen, für eine Erhöhung der Stoffaustauschrate betrieben.

Die spezifische Begasungsrate (Gasvolumenstrom) liegt vorzugsweise zwischen 0,1 und 10 vvm, besonders bevorzugt zwischen 0,5 und 5 vvm[-vvm bedeutet Volumen Gas/Volumen Bioreaktor/Minute]. Das Gas-Stripping wird vorzugsweise bei einem Druck zwischen 0,05 und 10 bar, besonders bevorzugt zwischen 0,5 und 1,3 bar durchgeführt. Ganz besonders bevorzugt wird das Gas-Stripping bei Unterdruck (bzw. negativem Überdruck) durchgeführt, d.h. bei einem Druck der niedriger ist als der Referenzdruck der Umgebung, welcher typischerweise ca. 1 bar beträgt. Bevorzugt erfolgt das Gas-Stripping bei Fermentationstemperatur. In einer alternativen, weiter bevorzugten Ausführungsform erfolgt das Gas-Stripping so, dass die Fermentationslösung zusätzlich erwärmt wird. Dies kann geschehen durch einen Aufbau, bei dem ein Teil der Fermentationslösung in eine externe Säule geleitet wird, in der die Temperatur erhöht ist und in der das Gas-Stripping stattfindet, wodurch das Gas-Stripping effizienter ist als bei Fermentationstemperatur.

Ein weiterer Vorteil des erfindungsgemäßen Verfahrens besteht darin, dass die durch den Übergang der flüchtigen Verbindungen von der Flüssigkeit in die Gasphase abgeführte Verdampfungsenthalpie zur Kühlung des Bioreaktors beiträgt und somit die erforderliche Kühlleistung zur Konstanthaltung der Temperatur im Bioreaktor verringert wird. In einer besonders bevorzugten Ausführungsform des erfindungsgemäßen Verfahrens ist keinerlei Kühlleistung mehr erforderlich, da die Summe aus abgeführter Verdampfungsenthalpie und Wärmeverlust an die Umgebung größer ist als die biologisch produzierte Wärme.

### c. Adsorption

Gemäß dem erfindungsgemäßen Verfahren wird der den Bioreaktor verlassende Gasstrom durch eine oder mehrere Säulen geleitet, welche mit einem oder mehreren Adsorbentien gefüllt sind. Als Adsorbentien eignen sich Zeolithe, Silica, Bentonite, Silicalite, Tone, Hydrotaicite, Aluminiumsilikate, Oxidpulver, Glimmer, Gläser, Aluminate, Clinoptolite, Gismondine, Quartze, Aktivkohlen, Knochenkohle, Montmorillonite, Polystyrole, Polyurethane, Polyacrylamide, Polymethacrylate und Polyvinylpyridine oder Mischungen davon. In einer bevorzugten Ausführungsform werden Zeolithe als Adsorbentien verwendet. Besonders bevorzugt sind Zeolithe des beta- oder MFI-Typs. Vorzugsweise weist der Zeolith. ein SiO₂/Al₂O₃-Verhältnis von 5 bis 1000 auf, und besonders bevorzugt ein SiO₂/Al₂O₃-Verhältnis von 100 bis 900. Besonders bevorzugt sind die synthetischen Zeolithe gemäß US 7,244,409.

Das Massenverhältnis von Adsorbens zu adsorbierten Ethanol liegt vorzugsweise zwischen 1 und 1000, besonders bevorzugt zwischen 2 und 20. Die Temperatur bei der Adsorption des Ethanols liegt vorzugsweise zwischen 10 und 100 °C, besonders bevorzugt zwischen 20 und 70 °C. Der Druck liegt vorzugsweise zwischen 0,5 und 10 bar, besonders bevorzugt zwischen 1 und 2 bar.

Das Adsorbermaterial kann in einer oder mehreren Säulen enthalten sein. Vorzugsweise werden mehrere, besonders bevorzugt 2 oder mehr, ganz besonders bevorzugt 2 bis 6 Säulen verwendet. Diese Säulen können in Reihe oder parallel geschaltet sein. Der Vorteil der Parallelschaltung ist, dass ein quasi-kontinuierlicher Betrieb ermöglicht wird, indem zwei oder mehr Säulen zwischen der Adsorption und der unter Punkt d näher beschriebenen Desorption alternieren, also Adsorption und Desorption simultan an unterschiedlichen Säulen durchgeführt werden können. Die Säulen werden vorzugsweise in einer Revolveranordnung bereitgestellt. In einer besonders bevorzugten Ausführungsform werden 2 bis 6 Säulen so geschaltet, dass die Säule bzw. Säulen, in denen die Adsorption abläuft, parallel zu der bzw. den Säulen geschaltet wird, in denen die Desorption abläuft. Läuft in mehr als einer Säule die Adsorption ab, so können diese Säulen in Reihe oder parallel geschaltet werden. So kann z.B. bei Verwendung von 6 Säulen in der "Revolver"-Konfiguration in den Säulen 1 bis 3 die Adsorption ablaufen, Säule 4 für die Desorption erwärmt werden, in Säule 5 die Desorption ablaufen, und Säule 6 lässt man auskühlen. Die Adsorptionsäule wird gewechselt, wenn die Beladung des Adsorbers einen vorher festgelegten Wert erreicht, spätestens jedoch wenn eine vollständige Beladung erreicht ist und die flüchtigen organischen Verbindungen am Säulenende durchbrechen, also nicht mehr vollständig adsorbiert werden können.

Typischerweise enthält der Gasstrom mehr Wasser als flüchtige organische Verbindungen, so dass sich die Adsorber zuerst mit Wasser sättigen. Die Beladung mit den flüchtigen organischen Verbindungen nimmt dann in einer zweiten Zeitspanne kontinuierlich zu, bis auch hier die Sättigung erreicht ist. In dieser zweiten Zeitspanne steigt das Verhältnis der flüchtigen organischen Verbindungen zu Wasser kontinuierlich an. Im Hinblick auf die nachfolgende katalytische Umsetzung besteht eine besonders bevorzugte Ausführungsform des Verfahrens darin, dass durch die Wahl einer geeigneten Taktzeit und/oder einer geeigneten Adsorbermenge dieses Verhältnis zwischen den flüchtigen organischen verbindungen und Wasser so eingestellt wird, dass es zu einem besonders geeigneten Mischungsverhältnis, d.h. zu einem besonders geeigneten oder optimalen Anteil der flüchtigen organischen Verbindungen für die katalytische Umsetzung kommt. Die dafür besonders günstigen oder optimalen Taktzeiten und/oder Adsorbermengen können durch Vorversuche ermittelt werden. Besonders geeignete Anteile flüchtiger organischer Verbindungen liegen zwischen 10 %(w/w) und 90 %(w/w), besonders bevorzugt zwischen 30 %(w/w) und 70 %(w/w) und noch bevorzugter zwischen 35 %(w/w) und 60 %(w/w). Die restlichen Anteile setzen sich aus Wasser und/oder Trägergas zusammen.

Das verwendete Adsorptionsmaterial ist bevorzugt zu selektiver Adsorption befähigt. Unter selektiver Adsorption an einem Adsorptionsmaterial wird dabei verstanden, dass das Adsorptionsmaterial aus einem Gasstrom einen höheren Massenanteil der erwünschten Verbindung als der unerwünschten Verbindung adsorbieren kann. Erwünschte Verbindungen im Sinne dieser Erfindung sind die flüchtigen organischen Verbindungen. Unerwünschte Verbindungen im Sinne dieser Erfindung sind, wie im nächsten Abschnitt spezifiziert, beispielsweise Katalysatorgifte wie Ammoniak. Das heißt, wenn der Gasstrom zu gleichen Massenanteilen aus der flüchtigen organischen Verbindung und der unerwünschten Verbindung besteht, mehr von der flüchtigen organischen Verbindung als von der unerwünschten Verbindung adsorbiert wird. Bevorzugt ist ein Verhältnis von flüchtiger organischer Verbindung zur unerwünschten Verbindung von mindestens 5:1, besonders bevorzugt von mindestens 20:1.

In einer bevorzugten Ausführungsform wird das Adsorbermaterial so ausgewählt, dass für die nachfolgende katalytische Umsetzung unerwünschte Verbindungen, wie zum Beispiel Katalysatorgifte, nur in vernachlässigbaren oder nicht messbaren Mengen adsorbiert werden. Typische unerwünschte Verbindungen, die allein oder in Kombination als Katalysatorgifte auftreten können, sind Ammoniak, Furane, Furfural sowie dessen Derivate wie Hydroxymethylfurfural (HMF), In einer besonders bevorzugten Ausführungsform wird die Adsorption von Ammoniak weitestgehend oder ganz vermieden, wenn ein Adsorbermaterial eingesetzt wird, das wenige Säurezentren aufweist. Geeignet sind hierfür zum Beispiel Zeolithe, die ein SiO₂/Al₂O₃-Verhältnis von mindestens 100 aufweisen. Diese zeolithe sind deshalb als Adsorbermaterial für diese Ausführungsform besonders bevorzugt.

Der an flüchtigen organischen Verbindungen abgereicherte Gasstrom kann nach Austritt aus der Adsorptionssäule zurück in den Bioreaktor geführt werden und steht dann erneut für das Gas-Stripping zur Verfügung. Die Adsorption kann im Wirbelschichtbetrieb durchgeführt werden. Ebenfalls können Radialadsorber oder Rotationsadsorber eingesetzt werden. Da der zurückgeführte Gasstrom in dieser Ausführungsform an organischen verbindungen abgereichert ist, kann die Konzentration der flüchtigen organischen Verbindungen im Fermentationsmedium trotz Gasrückführung niedrig gehalten werden.

Mit der erfindungsgemäßen Kombination aus in-situ-Gas-Stripping und Adsorption an Zeolith kann die Konzentration der flüchtigen organischen Verbindungen in der Fermentationslösung über die gesamte Fermentationsdauer unter einem bestimmten Wert gehalten werden. Dies ist besonders bevorzugt, wenn die flüchtigen organischen Verbindungen einen inhibierenden oder toxischen Effekt auf die Mikroorganismen ausüben, wie beispielsweise bei Ethanol, Butanol, oder Aceton. Die Adsorption wird vorzugsweise mindestens während der gesamten Dauer der Produktion der flüchtigen organischen Verbindungen durchgeführt, d.h. solange diese flüchtigen organischen Verbindungen entstehen. Eine niedrige Konzentration der flüchtigen organischen Verbindungen im Fermentationsmedium bedeutet beispielsweise weniger als 10 % (w/v) Gesamtmenge flüchtige organischen Verbindungen im Fermentationsmedium, bevorzugt weniger als 5 % (w/v) flüchtige organischen Verbindungen im Fermentationsmedium, besonders bevorzugt weniger als 3.5 % (w/v) flüchtige organischen Verbindungen im Fermentationsmedium, und am meisten bevorzugt weniger als 2 % (w/v) flüchtige organischen Verbindungen im Fermentationsmedium. In Bezug auf Einzelkomponenten ist bevorzugt weniger als 10 % (w/v) Ethanol, bevorzugt weniger als 5 % Ethanol im Fermentationsmedium, bzw. bevorzugt weniger als 3 % (w/v), bevorzugt weniger als 2 %, und noch bevorzugter weniger als 1,5 % (w/v) Butanol im Fermentationsmedium vorhanden, wobei Butanol im Sinne des in diesem Satz gesagten die Summe aller Butanole, also 1-Butanol, 2-Butanol und tertiär-Butanol umfasst.

### d. Desorption

Das erfindungsgemäße Verfahren ermöglicht die Desorption der flüchtigen organischen Verbindungen vom Adsorbens. Dabei liegt der Anteil flüchtiger organischer Verbindungen in Verfahrensschritt d. des erfindungsgemäßen Verfahrens in dem Desorbatstrom bevorzugt zwischen 10 % (w/w) und 90 % (w/w), besonders bevorzugt zwischen 30 % (w/w) und 70 % (w/w) und noch bevorzugter zwischen 35 % (w/w) und 60 %(w/w).

Die Desorption kann erfolgen durch Erhöhung der Temperatur und / oder Verringerung des Drucks innerhalb der Säule. Bevorzugt sind Temperaturen zwischen 25 und 300°C und Absolutdrücke zwischen 0 und 10 bar. Besonders bevorzugt sind Temperaturen zwischen 80 und 300°C, sowie Absolutdrücke zwischen 0,1 und 3 bar.

Bei einer bevorzugten Ausführungsform des erfindungsgemäßen Verfahrens wird für den Austrag der desorbierten flüchtigen organischen Verbindungen aus der Säule ein Trägergas verwendet. Besonders bevorzugt wird hierbei das gleiche inerte Trägergas verwendet, welches auch für das Gas-Stripping eingesetzt wird. In einer weiteren bevorzugten Ausführungsform des erfindungsgemäßen Verfahrens werden die Temperatur und der Absolutdruck des Trägergases entsprechend den oben beschriebenen Temperaturen und Absolutdrücken innerhalb der Säule eingestellt. Für diesen Zweck eignen sich vorgeschaltene Wärmetauscher und / oder Drosseln beziehungsweise Kompressoren.

Die Desorption kann im wirbelschichtbetrieb durchgeführt werden. Ebenfalls können Radialadsorber oder Rotationsadsorber eingesetzt werden.

### e. Katalytische Umsetzung

Entsprechend der vorliegenden Erfindung wird der in Abschnitt d beschriebene Desarbatstrom in einen oder mehrere mit Katalysator gefüllte Reaktoren überführt, wobei optional durch vorgeschaltete Wärmetauscher und Drosseln bzw. Kompressoren der Eingangsstrom auf Reaktionstemperatur und Reaktionsdruck gebracht werden kann. Abhängig von den gewählten Reaktionsbedingungen entstehen im Reaktor einzelne oder Gemische aus organischen Verbindungen, welche unter anderem den Gruppen der Olefine, Aliphate, Aromaten, Oxygenaten zugeordnet werden können.

Als Reaktoren können bevorzugt Wirbelschichtreaktoren, Radialstromreaktoren, Flugstromreaktoren, wanderbettreaktoren, Kreislaufreaktoren oder Festbettreaktoren eingesetzt werden. Diese Reaktoren sind im Rahmen der bevorzugten Ausführungsformen dieser Erfindung kurz beschrieben. Ebenso ist es möglich, mehrere Reaktoren gleicher oder unterschiedlicher Bauform zu kombinieren.

Als Katalysatoren eignen sich Brönsted und/oder Lewis-saure Stoffe, wie z.B. Zeolithe, Silica-Aluminas, Aluminas, mesoporöse Molekularsiebe, Hydroxyapatite, Bentonite, sulfatierte Zirkonias und Siliziumalumophosphate. In einer bevorzugten Ausführungsform werden Zeolithe als Katalysator verwendet. Bevorzugte Zeolithe sind Zeolithe des Typs MFI in H-Form (H-ZSM-5). Vorzugsweise verfügt der Zeolith über ein SiO₂/Al₂O₃-Verhältnis von 5 bis 1000, und besonders bevorzugt ein SiO₂/Al₂O₃-Verhältnis von 20 bis 200.

Für die katalytische Umsetzung bevorzugte Reaktionsbedingungen sind eine Temperatur von 150 bis 500°C, Absolutdrücke von 0,5 bis 100 bar und eine gas hourly space velocity (GHSV = Volumenstrom gasförmiges Edukt/Volumen Katalysator) von 100 bis 20000 h⁻¹. In einer besonders bevorzugten Ausführungsform liegt die Temperatur in einem Bereich von 250 bis 350°C, der Absolutdruck in einem Bereich von 1 bis 5 bar und die GHSV in einem Bereich von 2000 bis 8000 h⁻¹.

Ein Vorteil des erfindungsgemäßen Verfahrens gegenüber dem Stand der Technik liegt in der Kombination der im Abschnitt c/d beschriebenen Adsorption/Desorption mit der hier beschriebenen katalytischen Umsetzung. Durch gezielte Wahl der Adsorptions- bzw. Desorptionsbedingungen ist es erstmals möglich, den Wasseranteil sowie den Anteil der flüchtigen organischen Verbindungen im Desorbatstrom und somit im Eingangsstrom der katalytischen Umsetzung einzustellen. Durch geeignete Wahl des Anteils der flüchtigen organischen Verbindungen wird die Ausbeute an flüssigen organischen Verbindungen und durch den Wasseranteil das Deaktivierungverhalten des Katalysators signifikant beeinflusst.

Die katalytische Umsetzung erfolgt bevorzugt bei einer Temperatur von 150 bis 500°C, bevorzugt zwischen 250 und 350°C, einem Absolutdruck von 0,5 bis 100 bar, vorzugsweise zwischen 1 und 5 bar und einer GHSV von 100 bis 20000 h⁻¹, bevorzugt zwischen 2000 bis 8000 h⁻¹,

In einer bevorzugten Ausführungsform liegt der Anteil an flüchtigen organischen Verbindungen im Eingangsstrom bei 10 bis 90 % (w/w), in einer besonders bevorzugten Ausführungsform bei 30 bis 70 % (w/w) und in einer noch weiter bevorzugten Ausführungsform bei 35 bis 60 % (w/w). Die jeweils restlichen Anteile zu 100 % (w/w) setzen sich aus dem Anteil des Wassers bzw. dem Trägergas zusammen.

### f. Kondensation

Das erfindungsgemäße Verfahren kann in einer bevorzugten Ausführungsform weiterhin dadurch charakterisiert sein, dass im Anschluss an die oben beschriebenen Verfahrensschritte a bis e eine Kondensation des Produktstroms erfolgt, was optional durch Temperaturabsenkung und/oder Druckerhöhung geschehen kann. Bevorzugt ist dabei die Temperaturabsenkung auf ein Temperaturniveau unterhalb der Umgebungstemperatur, besonders bevorzugt unter 10°C. Für diese Abkühlung können Wärmetauscher eingesetzt werden, die im Gleichstrom, Gegenstrom oder im Kreuzstrom betrieben werden. Entsprechend einer bevorzugten Ausführungsform des erfindungsgemäßen Verfahrens erfolgt die Kondensation stufenweise, so dass mehrere Fraktionen mit unterschiedlichen Stoffzusammensetzungen erhalten werden.

Die vorliegende Erfindung umfasst auch ein Verfahren, das weiterhin dadurch charakterisiert ist, dass das Trägergas bzw. die Trägergase nach der Adsorption und/oder nach der katalytischen Umsetzung rückgeführt werden kann. Es ist bevorzugt, dass dabei die Fermentationsabgase als Trägergas eingesetzt werden. Die nicht kondensierbaren Anteile des Gasstroms werden vorzugsweise weiter katalytisch umgesetzt, vorzugsweise indem diese in die Säule der katalytischen Umsetzung rückgeführt werden.
Gemäß einer weiteren bevorzugten Ausführungsform des erfindungsgemäßen Verfahrens werden diese nicht kondensierbaren Anteile als Edukte für eine andere chemische Reaktion bzw. andere chemische Reaktionen, wie Polymerisationsreaktionen, verwendet. Besonders bevorzugt ist die Polymerisation von Ethylen zu Polyethylen oder von Propen zu Polypropylen. Gemäß einer weiteren bevorzugten Ausführungsform werden die nicht kondensierbaren Anteile thermisch verwertet, indem diese verbrannt werden. Bei allen diesen Ausführungsformen ist es auch möglich, zur Anreicherung der Komponenten eine weitere Adsorption mit folgender Desorption durchzuführen. Als Adsorbens wird dabei vorzugsweise ein zeolithisches Material eingesetzt. Besonders bevorzugt ist dabei das gleiche Material wie für die unter c und / oder e beschriebenen Verfahrensschritte einzusetzen.

Entsprechend dem erfindungsgemäßen Verfahren wird das anfallende Kondensat gesammelt. In einer bevorzugten Ausführungsform wird das anfallende Kondensat kühl gehalten, um einen Verlust durch Verdunstung zu vermeiden.

### g. Phasentrennung

Das unter f beschriebene Verfahren kann in einer weiter bevorzugten Ausführungsform weiterhin dadurch charakterisiert sein, dass im Anschluss an die. Kondensation eine Phasentrennung erfolgt. Aufgrund der Mischungslücke zwischen den organischen Verbindungen und Wasser bilden sich nach Kondensation bevorzugt zwei Phasen aus, eine organische und eine wässrige Phase. Gemäß dem erfindungsgemäßen Verfahren werden die Phasen voneinander getrennt. Dies kann durch einfaches Dekantieren oder aber durch Zentrifugation oder durch andere flüssig-flüssig-Trennverfahren erfolgen, die dem Fachmann bekannt sind. In einer besonders bevorzugten Ausführungsform werden bei der Dekantation die organischen Verbindungen als leichtere Phase, d.h. leichter als die wässrige Phase, abgetrennt. Ein besonderer Vorteil des erfindungsgemäßen Verfahrens besteht darin, dass somit eine große Menge Wasser ohne hohen energetischen Aufwand vom Produkt abgetrennt werden kann.

Die wässrige Phase kann als Prozesswasser in andere Verfahrensstufen zurückgeführt werden. Gemäß einer bevorzugten Ausführungsform wird die wässrige Phase mittels Gas-Stripping von eventuell noch darin gelösten flüchtigen Kohlenwasserstoffen befreit. Gemäß einer besonders bevorzugten Ausführungsform des Verfahrens werden diese flüchtigen Kohlenwasserstoffe rückgeführt und zwar entweder der Adsorption aus Abschnitt c oder der katalytischen Umsetzung aus Abschnitt e zugeführt, wobei als Trägergas-Strom entweder der gleiche Trägergas-Strom wie für das Gas-Stripping des Bioreaktors oder wie für die katalytische Umsetzung verwendet wird.

Die organische Phase kann entweder direkt oder nach einer weiteren Aufbereitung als Produkt gewonnen werden. Eine bevorzugte weitere Aufbereitung ist die Auftrennung des organischen Gemisches in mehrere Fraktionen und/oder Komponenten, welche jeweils unterschiedlich verwendet werden können.

Besonders vorteilhaft ist die Verwendung des Produktes oder Fraktionen davon als Kraftstoff oder als Zusatz für Kraftstoffe. Kraftstoffe können Ottokraftstoffe, Dieselkraftstoffe, Flugbenzine oder ähnliche Kraftstoffe sein. Überdies ist eine Verwendung des Produktes als Brennstoff möglich, beispielsweise als Heizöl. Eine alternative erfindungsgemäße Verwendung ist die weitere Nutzung für chemische Folgereaktionen, besonders bevorzugt zur Herstellung von Polymeren.

### Parallelschaltung

Das erfindungsgemäße Verfahren allgemein, so wie auch dessen Ausführungsformen, die zusätzlich die oben beschriebenen Schritte f und g enthalten, kann weiterhin dadurch charakterisiert sein, dass die Verfahrensschritte a bis e parallel ablaufen. Besonders bevorzugte, aber nicht limitierende Ausführungsformen hierzu sind im folgenden wiedergegeben:

### Besonders bevorzugte Ausführungsformen

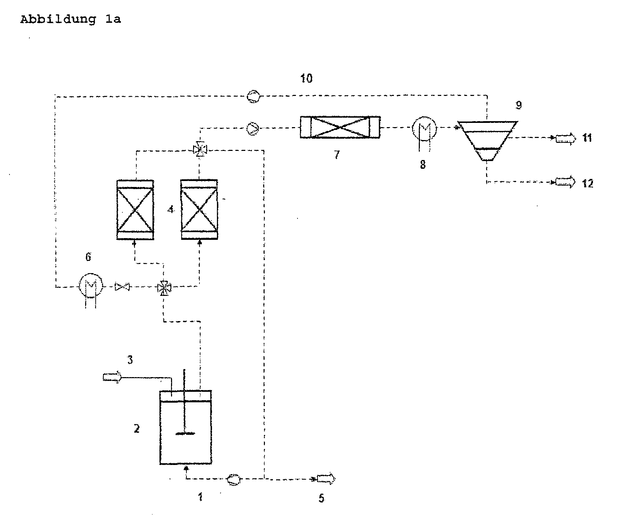

Abbildung 1a zeigt eine mögliche Ausführungsform des erfindungsgemäßen Verfahrens. Ein inerter Trägergasstrom (1) wird zum Gas-Stripping in den Bioreaktor (2) eingeblasen. Innerhalb des Bioreaktors wird Biomasse zu flüchtigen organischen Verbindungen fermentiert, wobei Hilfsstoffe (3) wie pH-Stellmittel zugegeben werden. Das den Bioreaktor verlassende Gas, welches flüchtige organische Verbindungen und andere flüchtige Bestandteile enthält, wird durch eine Adsorptionssäule (4) geleitet, in der die flüchtigen organischen Verbindungen selektiv adsorbiert werden. Der abgereicherte Gasstrom wird dann in den Bioreaktor zurückgeführt. Um einen quasi-kontinuierlichen Betrieb zu gewährleisten, werden zwei oder mehrere Säulen parallel und/oder in Reihe geschaltet. Ein Teil des Trägergasstroms wird aufgrund der fermentativ gebildeten Fermentationsabgase abgeführt (5). Zur Desorption der adsorbierten organischen Verbindungen wird die Temperatur und / oder der Druck innerhalb der Säulen (4) verändert. Der für den Austrag der desorbierten flüchtigen organischen Verbindungen notwendige Trägergasstrom (10) wird über einen Wärmetauscher (6) und / oder Drosseln entsprechend eingestellt.

Das bei der Desorption die Säule verlassende Gas wird anschließend in einem oder mehreren Reaktoren (7) katalytisch umgesetzt. Die dabei gebildeten organischen Produkte werden über einen Wärmetauscher (8) kondensiert. Das Kondensat wird dann einer Phasentrennung (9) unterzogen. Die organische Phase wird als Produkt (11) abgeführt und die wässrige Phase (12) kann weiteren Verwendungen zugeführt werden. Der regenerierte Trägergasstrom (10) wird rückgeführt.

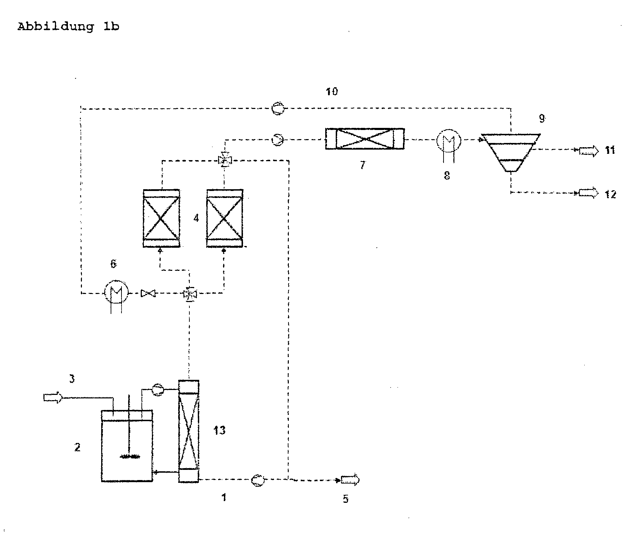

Abbildung 1b zeigt eine weitere mögliche Ausführungsform des erfindungsgemäßen verfahrens, wobei in diesem Fall das Gas-Stripping in einer mit dem Bioreaktor verbundenen externen Gas-Stripping-Säule (13) erfolgt. Dabei wird Fermentationslösung der externen Gas-Stripping-Säule zugeführt und die gestrippte Lösung dann wieder zurück in den Bioreaktor geführt. Alle weiteren Verfahrensschritte sind analog zu Abbildung 1a.

Entsprechend dem erfindungsgemäßen Verfahren wird für die Adsorption und die katalytische Umsetzung in einer besonders bevorzugten Ausführungsform als Träger und Katalysator das gleiche Aktivmaterial verwendet. Dadurch sind folgende weitere, besonders bevorzugte Ausführungsformen des erfindungsgemäßen Verfahrens möglich:

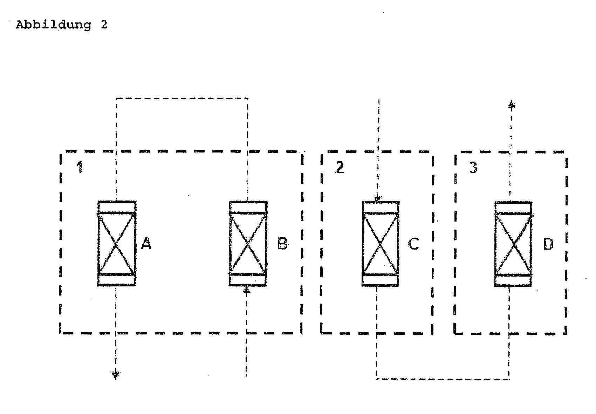

Abbildung 2 zeigt eine weitere mögliche Ausführungsform des erfindungsgemäßen Verfahrens: die Revolver-Lösung, bei der vier Säulen (A-D) oder mehr eingesetzt werden. Zunächst sind die Säulen A und B in der Adsorption (1), wobei die Säulen in Reihe, aber auch parallel geschaltet sein können. Säule C befindet sich in der Desorption (2), indem ein Trägergasstrom bei erhöhten Temperaturen oder reduziertem Druck eingeblasen wird. In Säule D erfolgt die katalytische Umsetzung, wobei der desorbierte Gasstrom eingeblasen wird. Nach Ende der Taktzeit gelangt Säule B in die Desorption (2), C in die katalytische Umsetzung (3) und D in die Adsorption (1). Für die Adsorption sind dann die Säulen D und A geschaltet. Nach so vielen Taktzeiten wie vorhandenen Säulen wird wieder dieselbe Säule desorbiert wie zu Beginn, so dass ein Zyklus vollendet ist und ein neuer beginnt.

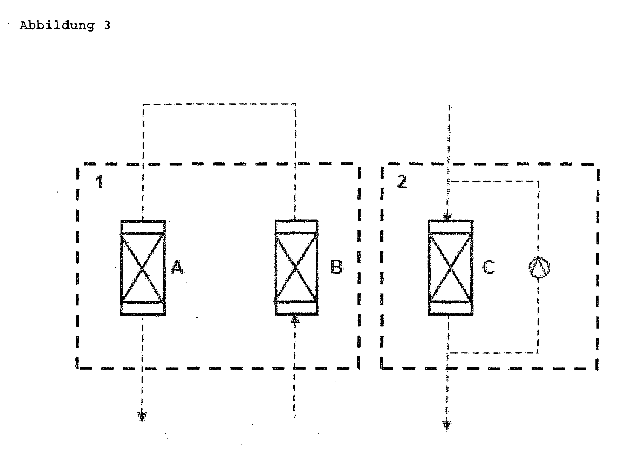

Abbildung 3 zeigt eine weitere mögliche Ausführungsform des erfindungsgemäßen Verfahrens, bei der drei Säulen (A-C) oder mehr eingesetzt werden und bei der die Desorption und die katalytische Umsetzung zeitgleich in derselben Säule stattfinden. Zunächst sind die Säulen A und B in der Adsorption (1), wobei die Säulen in Reihe, aber auch parallel geschaltet sein können. In Säule C werden über Temperaturerhöhung die flüchtigen organischen Verbindungen desorbiert und zugleich katalytische umgesetzt (3). Um eine bestimmte Verweilzeitverteilung einstellen zu können, wird ein Teil des Desorbatgasstroms in Säule C rückgeführt. Nach Ende der Taktzeit gelangt Säule B in die Desorption und katalytische Umsetzung (2) und C in die Adsorption (1). Für die Adsorption sind dann die Säulen C und A geschaltet. Nach so vielen Taktzeiten wie vorhandenen Säulen wird wieder in derselben Säule adsorbiert wie zu Beginn, so dass ein Zyklus vollendet ist und ein neuer beginnt.

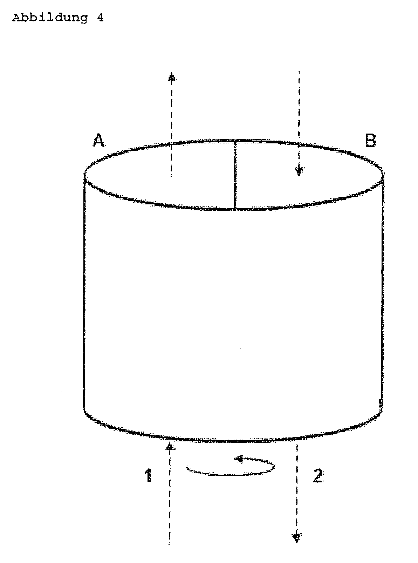

Abbildung 4 zeigt eine weitere mögliche Ausführungsform des erfindungsgemäßen Verfahrens unter Verwendung eines Radialadsorbers, der aus zwei Zonen besteht. In Zone A erfolgt die Adsorption aus dem Gasstrom (1), der die flüchtigen organischen Verbindungen enthält, in Zone B die Desorption und gleichzeitige katalytische Umsetzung zum Produktgasstrom (2). Durch Rotation des Apparats gelangt kontinuierlich beladenes Adsorptionsmaterial von der Adsorptionszone (A) in die Desorptions- und katalytische Umsetzungszone (B) und umgekehrt.

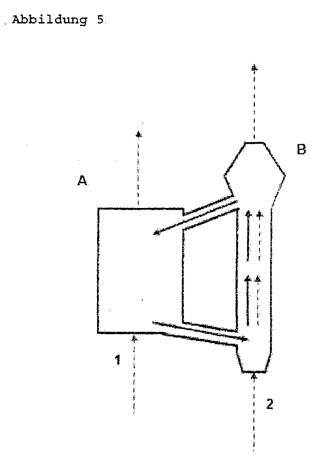

Abbildung 5 zeigt eine weitere mögliche Ausführungsform des erfindungsgemäßen Verfahrens unter Verwendung eines Flugstromreaktors, der über eine Adsorptionszone (A) und eine Reaktionszone (B) verfügt. In der Adsorptionszone (A) erfolgt die Adsorption der flüchtigen organischen Verbindungen aus dem Gasstrom (1), in Zone B erfolgt durch Einblasen eines heißen Trägergasstroms (2) die Desorption und katalytische Umsetzung, wobei der heiße Trägergasstrom die Partikel mitreißt und nach oben innerhalb des sogenannten Risers befördert. Dabei werden Gas (gestrichelte Linie) und Partikel (durchgezogene Linie) im Gleichstrom gefördert. Am Kopf des Risers erfolgt eine Partikelabscheidung. Die Partikel gelangen dann zurück in die Adsorptionszone (A), so dass insgesamt eine Kreislaufführung der Partikel entsteht.

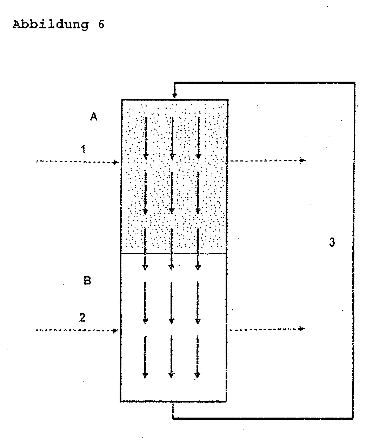

Abbildung 6 zeigt eine weitere mögliche Ausführungsform des erfindungsgemäßen Verfahrens unter Verwendung eines Wanderbettreaktors, der über eine Adsorptionszone (A) und eine Reaktionszone (B) verfügt. In der kühleren Adsorptionszone (A) erfolgt die Adsorption der flüchtigen organischen Verbindungen aus dem Trägergasstrom (1). Die beladenen Partikel wandern dann in die wärmere Reaktionszone (B), in der die Desorption und die katalytische Umsetzung erfolgen. Mit Hilfe eines Trägergasstroms (2) werden die organischen Produkte aus dem Reaktor ausgeschleust. Die Partikel werden nach der Reaktionszone aus dem Reaktor befördert und über geeignete Feststofffördertechniken wieder in die Adsorptionszone (A) gefördert), so dass insgesamt eine Kreislaufführung der Partikel entsteht.

In einer weiteren bevorzugten Ausführungsform ist das Verfahren gemäß dieser Erfindung weiterhin dadurch charakterisiert, dass eine, vorzugsweise zwei, noch bevorzugter drei, noch bevorzugter vier, noch bevorzugter fünf oder mehr der einzelnen Verfahrensschritte unter folgenden Bedingungen durchgeführt werden:
a. die Fermentation erfolgt bei Temperaturen zwischen 10 und 70°C, bevorzugt zwischen 20 und 60°C, besonders bevorzugt zwischen 30 und 50°C,
b. beim Gas-Stripping liegt die spezifische Begasungsrate zwischen 0,1 und 10 vvm, vorzugsweise zwischen 0,5 und 5 vvm,
c. die Temperatur bei der Adsorption liegt zwischen 10 und 100°C, vorzugsweise zwischen 20 und 70°C und der Druck zwischen 0,5 und 10 bar, vorzugsweise zwischen 1 und 2 bar,
d. die Desorption erfolgt über Temperaturerhöhung und/oder Druckabsenkung,
e. die katalytische Umsetzung erfolgt bei einer Temperatur von 150 bis 500°C, bevorzugt zwischen 250 und 350°C, einem Absolutdruck von 0,5 bis 100 bar, vorzugsweise zwischen 1 und 5 bar und einer GHSV von 100 bis 20000 h⁻¹, bevorzugt zwischen 2000 bis 8000 h⁻¹,
f. die Kondensation erfolgt über Temperaturabsenkung und/oder Druckerhöhung,
g. bei der Dekantation werden die organischen Verbindungen als leichtere Phase abgetrennt.

Es ist erfindungsgemäß möglich, die im vorangehenden Abschnitt aufgeführten Bedingung (en) mit der Verwendung eines der bevorzugten Reaktoren gemäß Abbildungen 1 bis 6 zu kombinieren.

### Kurze Beschreibung der Abbildungen.

Abbildung 1 (a und b) zeigt Ausführungsbeispiele des erfindungsgemäßen Verfahrens mit Gas-Stripping im Bioreaktor (1a) und mit Gas-Stripping in einer externen Gas-Stripping-Säule (1b).

Abbildung 2 zeigt eine erfindungsgemäße Ausführungsform mit Revolverkonfiguration.

Abbildung 3 zeigt eine erfindungsgemäße Ausführungsform mit Rückführung des Desorbatgasstroms in dieselbe Säule.

Abbildung 4 zeigt eine erfindungsgemäße Ausführungsform mit einem Radialadsorber.

Abbildung 5 zeigt eine erfindungsgemäße Ausführungsform mit einem Flugstromreaktor.

Abbildung 6 zeigt eine erfindungsgemäße Ausführungsform mit einem Wanderbettreaktor.

Abbildung 7 zeigt die Einstellung des Ethanolanteils und des Wasseranteils durch unterschiedliche Adsorptionstemperaturen gemäß Beispiel 1.

Abbildung 8 zeigt den Vergleich zweier Fermentationen mit *Pachysolen tannophilus* ohne (oben) und mit kontinuierlicher Abtrennung von Ethanol über Gas-Stripping und Adsorption gemäß Beispiel 2 (unten; die kurz-gestrichelte Summen-Ethanolkurve berücksichtigt die Summe aus Ethanol in der Lösung und auf dem Adsorber gebunden).

Abbildung 9 zeigt den Einfluss des Ethanolanteils im gasförmigen Desorbatstrom auf die Ausbeute der flüssigen organischen Phase, bezogen auf die eingesetzte Menge Ethanol gemäß Beispiel 3.

### Beispiele

### Beispiel 1: Gas-Stripping und Adsorption bei unterschiedlichen Temperaturen

500 mL einer 5% (w/v)-Ethanol-Wasser-Lösung wurden für 24 Stunden mit einem Volumenstrom von 1 L/min gestrippt. Es wurden eine Membranpumpe (KNF, Deutschland), ein Volumenstromregler (Swagelok, Deutschland) und eine Gaswaschflasche (VWR, Deutschland) verwendet. Der Gasstrom wurde durch eine Glassäule (VWR, Deutschland) geleitet, welche mit 200 g Zeolith-Granulat (ZSM-5, H-Form; SiO₂/Al₂O₃=200; Binder: Bentonit; Durchmesser 2-4 mm; Hersteller: Süd-Chemie AG, Deutschland) gepackt war. Der Gasstrom wurde im Rahmen einer Kreislaufführung in die Gaswaschflasche rückgeführt, so dass das System geschlossen war. Die Glassäule wurde über eine Heizmanschette (Mohr & Co GmbH, Deutschland) auf verschiedene Temperaturen erwärmt. Das Gas-Stripping in der Gaswaschflasche erfolgte bei 30°C. Nach Versuchsende wurde die Ethanol-Konzentration über Gas-Chromatografie (Trace GC, ThermoFischer, Deutschland) in der Lösung bestimmt. Zudem wurde die Gewichtszunahme des Zeolithen und der Lösung bestimmt. Über eine Massenbilanz wurden dann die Beladungen des Zeolithen mit Wasser und Ethanol und daraus der Wasseranteil und der Anteil der flüchtigen organischen Verbindung Ethanol berechnet.

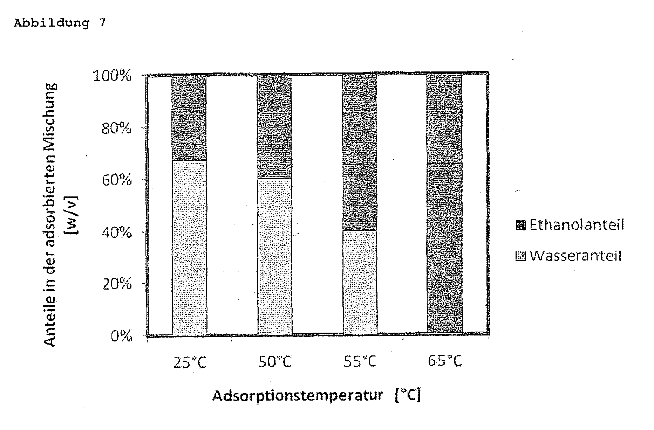

Abbildung 7 zeigt die erhaltenen Wasseranteile und die Anteile der flüchtigen organischen Verbindungen in Abhängigkeit von der Adsorptionstemperatur. Dementsprechend kann der Wasseranteil bzw. der Anteil der flüchtigen organischen Verbindungen über die Adsorptionstemperatur eingestellt werden. *Beispiel 2: In-situ-Fermentation mit Gas-Stripping und Adsorption*

*Pachysolen tannophilus* (DSM 70352, DSMZ, Braunschweig) wurde mit und ohne kontinuierlicher Abtrennung von Ethanol über Gas-Stripping und Adsorption unter ansonsten identischen Bedingungen für 100 Stunden bei 30°C und pH 6 fermentiert. Als Substrat wurde vorbehandelte und hydrolysierte lignocellulosische Biomasse eingesetzt, die ca. 70 g/L Glucose und ca. 30 g/L Xylose enthielt. Als Bioreaktor wurden Bioreaktor mit einem Füllvolumen von jeweils 0,8 L verwendet. Bei der Fermentation mit kontinuierlicher Abtrennung erfolgte das Gas-Stripping mit einer spezifischen Begasungsrate von 1 vvm mit Hilfe einer Membranpumpe (KNF, Deutschland). Wie in Beispiel 1 wurde der Gas-Strom durch eine Glassäule geleitet und dann rückgeführt. Die Glassäule war mit 535 g Zeolith-Granulat (ZSM-5, H-Form; SiO₂/Al₂O₃=200; Binder: Bentonit; Durchmesser 2-4 mm; Hersteller: Süd-Chemie AG, Deutschland) gepackt. Während der Fermentation wurden Proben genommen und der Ethanol-Gehalt gaschromatografisch und die Zucker über HPLC quantifiziert. Zudem wurde die Gewichtszunahme des Zeolithen und der Wasseranteil der adsorbierten Mischung über Karl-Fischer-Titration (Schott Instruments, Deutschland) bestimmt. Aus Vorversuchen ist bekannt, dass lediglich Wasser und Ethanol unter den gegebenen Bedingungen adsorbiert werden. Dadurch kann aus dem Wassergehalt auf den Ethanolanteil rückgeschlossen werden.

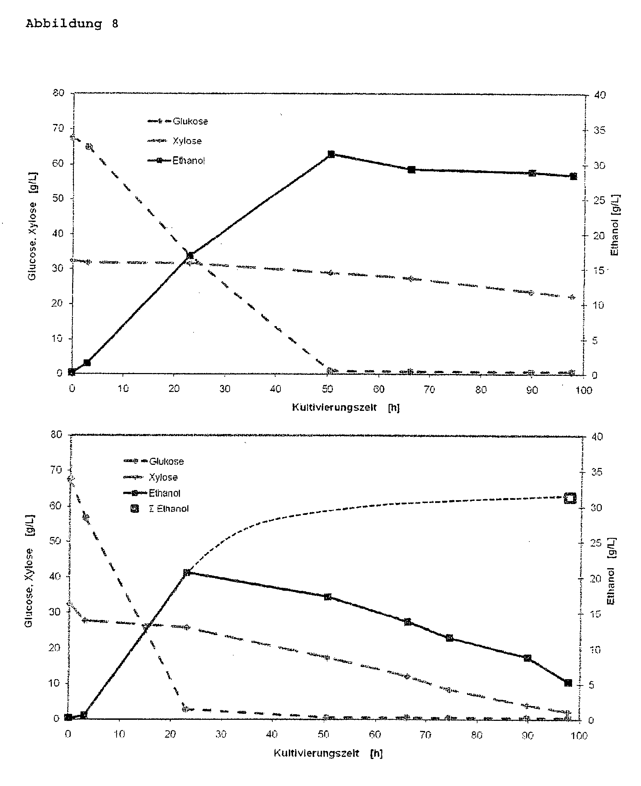

Abbildung 8 zeigt die erhaltenen Konzentrationsverläufe. Dabei wird ersichtlich, dass die simultane Durchführung von Fermentation, Gas-Stripping und Adsorption vorteilhaft ist und dass unter den gegebenen Bedingungen die Fermentation mit kontinuierlicher Abtrennung der flüchtigen Verbindungen höhere Raum-Zeit-Ausbeuten erzielt werden.

### Beispiel 3: Katalytische Umsetzung

Für die katalytische Umsetzung wurde ein Festbettreaktor (Länge=50 cm, Innendurchmesser=2,5 cm) der Firma ILS - Integrated Lab Solutions GmbH verwendet. Das flüssige Modell-Desorbat (40 Gew.-% EtOH, 60 Gew.-% Wasser) wurde mit einer HPLC-Pumpe (Smartline Pump 100, Wissenschaftliche Gerätebau Dr. Ing. Herbert Knauer GmbH) in das Reaktionsrohr dosiert, wo es mittels einer beheizten vorpackung aus inertem SiC verdampft, mit Stickstoff vermischt, so dass 4 Gew.-% Stickstoff anwesend waren, und auf die Reaktionstemperatur von 300°C und den Absolutdruck von 3 bar gebracht wurde. Der so erhaltene gasförmige Desorbatstrom wurde schließlich mit einer gas hourly space velocity (GHSV) von 5800 h⁻¹ über eine Schüttung aus 10 g Zeolith-Extrudat (Zeolith ZSM-5, H-Form, SiO₂/Al₂O₃=90; Binder Al₂O₃; Durchmesser=1/16 inch; Hersteller: Süd-Chemie AG) geleitet. Der gasförmige Produktstrom wurde in einem dem Festbettreaktor nachgeschaltetem Gas/Flüssig-Separator auf 10°C abgekühlt, wobei die flüssigen Produkte kondensiert und von den gasförmigen Produkten getrennt wurden. Anschließend wurde die flüssige organische Phase von der wässrigen Phase abdekantiert. Der Versuch wurde insgesamt über eine time-on-stream (TOS) von 24 h durchgeführt.
Die während dieser Zeitspanne angefallene flüssige organische Phase wurde abschließend durch Gas-Chromatographie mit gekoppelter Massenspektrometrie analysiert (Zusammensetzung siehe Tabelle 1). Wie die Auswertung zeigte, wurde unter diesen Versuchsbedingungen ein Ethanolumsatz von >99 % und eine Ausbeute der flüssigen organische Phase, bezogen auf die eingesetzte Menge Ethanol, von 34 Gew.-% erreicht.

**Tabelle 1: Zusammensetzung der flüssigen organischen Phase**

| ***Substanzklasse*** | ***Anteil [GC Area %]*** |
|---|---|
| unverzweigte Alkane (<C5) | 1,6 |
| unverzweigte Alkane (C5-C10) | 3,8 |
| verzweigte Alkane (C5-C10) | 30,3 |
| verzweigte Olefine (C5-C10) | 2,7 |
| cyclische Kohlenwasserstoffe | 2,9 |
| Benzol | 0,3 |
| Toluol | 3,8 |
| Xylole | 10,8 |
| einfach alkylierte Aromate (ohne Toluol, Xylole) | 43, 8 |

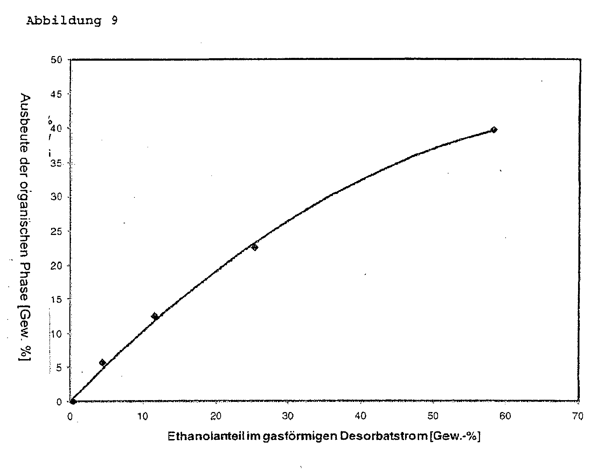

In einem weiteren Experiment wurde unter ansonsten identischen Bedingungen der Ethanolanteil im gasförmigen Desorbatstrom variiert, indem unterschiedliche Modell-Desorbate in der beheizten Vorpackung verdampft und mit unterschiedlichen Stickstoffmengen gemischt wurden. Abbildung 9 zeigt den Einfluss des Ethanolanteils im gasförmigen Desorbatstrom auf die Ausbeute der flüssigen organische Phase, bezogen auf die eingesetzte Menge Ethanol. Es ist zu erkennen, dass sich ein hoher Ethanolanteil vorteilhaft auf die Ausbeute der flüssigen organischen Phase auswirkt.

## Patentansprüche

1. Verfahren zur Herstellung von organischen Verbindungen umfassend die nachfolgenden Verfahrensschritte:
a. die fermentative Umsetzung von Biomasse zu flüchtigen organischen Verbindungen in einem Bioreaktor;
b. die Entfernung der flüchtigen organischen Verbindungen durch Gas-Stripping mit Hilfe eines Trägergases;
c. die Adsorption der flüchtigen organischen Verbindungen aus dem Gasstrom;
d. die Desorption der adsorbierten flüchtigen organischen Verbindungen von dem Adsorber;
e. die katalytische Umsetzung der flüchtigen organischen Verbindungen.

2. Verfahren nach Anspruch 1, wobei in Verfahrensschritt d der Anteil flüchtiger organischer Verbindungen in dem Desorbatstrom zwischen 10 % (w/w) und 90 % (w/w) liegt.

3. Verfahren nach Anspruch 2, wobei im Anschluss an die Verfahrensschritte a bis e eine Kondensation des Produktstroms erfolgt.

4. Verfahren nach einem der Ansprüche 1 bis 3, wobei die Verfahrensschritte a bis e parallel ablaufen.

5. Verfahren nach einem der Ansprüche 1 bis 4, wobei es sich bei den flüchtigen organischen Verbindungen um Alkohole und/oder Ketone und/oder Aldehyde und/oder organische Säuren handelt.

6. Verfahren nach einem oder mehreren der vorhergehenden Ansprüche, wobei das Trägergas bzw. die Trägergase nach der Adsorption und/oder nach der katalytischen Umsetzung rückgeführt werden.

7. Verfahren nach einem oder mehreren der Ansprüche 3 bis 6, weiterhin dadurch charakterisiert, dass ein oder mehr der einzelnen Verfahrensschritte unter folgenden Bedingungen durchgeführt werden:
a. die Fermentation erfolgt bei Temperaturen zwischen 10 und 70°C,
b. beim Gas-Stripping liegt die spezifische Begasungsrate zwischen 0,1 und 10 vvm,
c. die Temperatur bei der Adsorption liegt zwischen 10 und 100°C und der Druck zwischen 0,5 und 10 bar,
d. die Desorption erfolgt über Temperaturerhöhung und/oder Druckabsenkung,
e. die katalytische Umsetzung erfolgt bei einer Temperatur von 150 bis 500°C, einem Absolutdruck von 0,5 bis 100 bar und einer GHSV von 100 bis 20000 h⁻¹,
f. die Kondensation erfolgt über Temperaturabsenkung und/oder Druckerhöhung,
g. bei der Dekantation werden die organischen Verbindungen als leichtere Phase abgetrennt.

8. Verfahren nach einem oder mehreren der vorhergehenden Ansprüche, wobei der Adsorber ein Zeolith ist.

9. Verfahren gemäß einem oder mehreren der vorhergehenden Ansprüche, wobei der Katalysator ein Zeolith ist.

10. Verfahren nach Anspruch 1, wobei der Adsorber ein Zeolith ist, der ein SiO₂/Al₂O₃-Verhältnis von mindestens 100 aufweist.

11. Verfahren nach einem oder mehreren der Ansprüche 1 bis 9, wobei es sich beim Zeolith-Adsorber und beim Zeolith-Katalysator um das identische Material handelt.

12. Verfahren nach Anspruch 11, wobei das Zeolith-Material in mehrere parallele Säulen gefüllt ist, die im Sinne einer Revolverkonfiguration zeitlich voneinander versetzt zwischen mehreren Verfahrensschritten wechseln, wobei diese Verfahrensschritte ausgewählt werden aus Adsorption, Desorption, katalytischer Umsetzung sowie möglicherweise Regeneration.

13. Verfahren nach Anspruch 11, wobei Adsorption, Desorption und katalytische Umsetzung zeitlich versetzt in jeweils der gleichen Säule stattfinden.

14. Verfahren nach Anspruch 11, wobei Adsorption, Desorption und katalytische Umsetzung in einem einzigen Apparat erfolgt.

15. Verfahren nach Anspruch 14, wobei es sich bei dem Apparat um einen Radialadsorber, Wanderbettreaktor oder um einen Flugstromreaktor handelt.

## Claims

1. Method for the manufacture of organic compounds comprising the following steps:
a) the fermentative conversion of biomass to volatile organic compounds in a bioreactor;
b) removal of the volatile organic compounds by gas stripping using a carrier gas;
c) adsorption of the volatile organic compounds from the gas stream;
d) desorption of the adsorbed volatile organic compounds from the adsorber;
e) catalytic conversion of the volatile organic compounds.

2. Method according to claim 1, wherein in step d) the percentage of volatile organic compounds in the desorbate stream is between 10% (w/w) and 90% (w/w).

3. Method according to claim 2, wherein following steps a) to e), condensation of the product stream is effected.

4. Method according to any of claims 1 to 3, wherein steps a) to e) run in parallel.

5. Method according to any of claims 1 to 4, wherein the volatile organic compounds are alcohols and/or ketones and/or aldehydes and/or organic acids.

6. Method according to one or more of the preceding claims, wherein the carrier gas or gases are recirculated after adsorption and/or after catalytic conversion.

7. Method according to one or more of claims 3 to 6, further **characterised in that** one or more of the individual steps are performed under the following conditions:
a) fermentation is effected at temperatures of between 10 and 70°C,
b) during gas stripping, the specific gassing rate is between 0.1 and 10 vvm,
c) the temperature during adsorption is between 10 and 100°C and the pressure between 0.5 and 10 bars,
d) desorption is effected by increasing the temperature and/or decreasing the pressure,
e) catalytic conversion is effected at a temperature from 150 to 500°C, an absolute pressure from 0.5 to 100 bars and a GHSV from 100 to 20,000 h^{-I},
f) condensation is effected by decreasing the temperature and/or increasing the pressure,
g) during decantation, the organic compounds are separated off as a lighter phase.

8. Method according to one or more of the preceding claims, wherein the adsorber is a zeolite.

9. Method according to one or more of the preceding claims, wherein the catalyst is a zeolite.

10. Method according to claim 1, wherein the adsorber is a zeolite which has a SiO₂/Al₂O₃ ratio of at least 100.

11. Method according to one or more of claims 1 to 9, wherein the zeolite adsorber and the zeolite catalyst are the identical material.

12. Method according to claim 11, wherein the zeolite material is poured into several parallel columns which change between several steps with a time lag according to a turret configuration, wherein these steps are selected from adsorption, desorption, catalytic conversion and possibly regeneration.

13. Method according to claim 11, wherein adsorption, desorption and catalytic conversion take place with a time lag in each case in the same column.

14. Method according to claim 11, wherein adsorption, desorption and catalytic conversion are effected in a single apparatus.

15. Method according to claim 14, wherein the apparatus is a radial adsorber, moving-bed reactor or an entrained-flow reactor.

## Revendications

1. Procédé de production de composés organiques comprenant les étapes de procédé suivantes :
a. la conversion par fermentation de biomasse en composés organiques volatils dans un bioréacteur ;
b. l'élimination des composés organiques volatils par stripage au gaz à l'aide d'un gaz support ;
c. l'adsorption des composés organiques volatils à partir du courant gazeux ;
d. la désorption des composés organiques volatils adsorbés depuis l'adsorbeur ;
e. la conversion catalytique des composés organiques volatils.

2. Procédé selon la revendication 1, dans lequel, à l'étape de procédé d, la proportion de composés organiques volatils dans le courant du désorbat se situe entre 10 % (w/w) et 90 % (w/w).

3. Procédé selon la revendication 2, dans lequel, à la suite des étapes de procédé a à e, il se produit une condensation du courant de produits.

4. Procédé selon une des revendications 1 à 3, dans lequel les étapes de procédé a à e se déroulent en parallèle.

5. Procédé selon une des revendications 1 à 4, dans lequel les composés organiques volatils sont des alcools et/ou des cétones et/ou des aldéhydes et/ou des acides organiques.

6. Procédé selon une ou plusieurs des revendications précédentes, dans lequel le gaz véhicule ou les gaz véhicules est/sont renvoyé(s) dans le circuit après l'adsorption et/ou après la conversion catalytique.

7. Procédé selon une ou plusieurs des revendications 3 à 6, **caractérisé en outre en ce qu'**une ou plus d'une étape de procédé individuelle sont réalisées dans les conditions suivantes :
a. la fermentation s'effectue à des températures situées entre 10 et 70°C,
b. le taux de gazage spécifique se situe entre 0,1 et 10 vvm lors du stripage au gaz,
c. la température lors de l'adsorption se situe entre 10 et 100°C et la pression entre 0,5 et 10 bars,
d. la désorption s'effectue lors d'une hausse de la température et/ou d'une baisse de la pression,
e. la conversion catalytique s'effectue à une température de 150 à 500°C, une pression absolue de 0,5 à 100 bars et une VSHG (vitesse spatiale horaire des gaz) de 100 à 20000 h⁻¹,
f. la condensation s'effectue lors d'une baisse de température et/ou d'une hausse de la pression,
g. lors de la décantation, les composés organiques sont séparés sous forme de phase plus légère.

8. Procédé selon une ou plusieurs des revendications précédentes, dans lequel l'adsorbeur est une zéolithe.

9. Procédé selon une ou plusieurs des revendications précédentes, dans lequel le catalyseur est une zéolithe.

10. Procédé selon la revendication 1, dans lequel l'adsorbeur est une zéolithe qui présente un rapport SiO₂/Al₂O₃ d'au moins 100.

11. Procédé selon une ou plusieurs des revendications 1 à 9, dans lequel l'adsorbeur zéolithique et le catalyseur zéolithique sont le même matériau.

12. Procédé selon la revendication 11, dans lequel le matériau zéolithique est chargé dans plusieurs colonnes parallèles qui sont alternées entre plusieurs étapes de procédé de manière décalée dans le temps au sens d'une configuration en revolver, ces étapes de procédé étant choisies parmi l'adsorption, la désorption, la conversion catalytique et une éventuelle régénération.

13. Procédé selon la revendication 11, dans lequel l'adsorption, la désorption et la conversion catalytique se produisent de manière décalée dans le temps dans la même colonne à chaque fois.

14. Procédé selon la revendication 11, dans lequel l'adsorption, la désorption et la conversion catalytique se font dans un unique appareil.

15. Procédé selon la revendication 14, dans lequel l'appareil est un adsorbeur radial, un réacteur en lit fluidisé ou un réacteur en flux aérien.
